# EUROPEAN PATENT APPLICATION

(11) **EP 2 388 012 A1**
(43) Date of publication of application: **23.11.2011**
(21) Application number: 10163479.8
(22) Date of filing: 20.05.2010
(51) Int. Cl.: A61K 38/04, A61K 38/07, A61K 38/08, A61K 38/17, C07K 2/00, C07K 5/10, C07K 7/06, A61P 3/10, A61P 25/28

(54) **Kisspeptin peptides for use in the treatment of Alzheimer's disease, Creutzfeldt-Jakob disease or diabetes mellitus**

(71) Applicant: Roehampton University, London SW15 5PJ (GB)
(72) Inventor: Milton, Nathaniel, Surrey, Surrey KT4 7RX (GB)
(74) Representative: Jappy, John William Graham

(57) **Abstract**

A peptide comprises the amino acid sequence defined herein as SEQ ID No. 2, for use in the treatment of a condition associated with Amyloid fibril-forming peptides, wherein the peptide does not directly activate or inhibit the GPR-54 G-protein coupled receptor.

SEQUENCE LISTING

<110>  Roehampton University

<120>  Peptides for Therapeutic Use

<130>  JWJ01629EP

<160>  12

<170>  PatentIn version 3.3

<210>  1
<211>  54
<212>  PRT
<213>  Homo sapiens

<400>  1

Gly Thr Ser Leu Ser Pro Pro Pro Glu Ser Ser Gly Ser Arg Gln Gln
1               5                   10                  15

Pro Gly Leu Ser Ala Pro His Ser Arg Gln Ile Pro Ala Pro Gln Gly
            20                  25                  30

Ala Val Leu Val Gln Arg Glu Lys Asp Leu Pro Asn Tyr Asn Trp Asn
        35                  40                  45

Ser Phe Gly Leu Arg Phe
    50

<210>  2
<211>  6
<212>  PRT
<213>  Homo sapiens

<400>  2

Tyr Asn Trp Asn Ser Phe
1               5

<210>  3
<211>  42
<212>  PRT
<213>  Homo sapiens

<400>  3

<210>  4
<211>  13
<212>  PRT
<213>  Homo sapiens

<400>  4

Leu Pro Asn Tyr Asn Trp Asn Ser Phe Gly Leu Arg Phe
1               5                   10

<210>  5
<211>  10
<212>  PRT
<213>  Homo sapiens

<400>  5

Tyr Asn Trp Asn Ser Phe Gly Leu Arg Phe
1               5                   10

<210>  6
<211>  10
<212>  PRT
<213>  Homo sapiens

<400>  6

Asp Leu Pro Asn Tyr Asn Trp Asn Ser Phe
1               5                   10

<210>  7
<211>  37
<212>  PRT
<213>  Homo sapiens

<400>  7

<210>  8
<211>  30

<212>  PRT
<213>  Homo sapiens

<400>  8

Lys Thr Asn Met Lys His Met Ala Gly Ala Ala Ala Ala Gly Ala Val
1               5                   10                  15

Val Gly Gly Leu Gly Gly Tyr Met Leu Gly Ser Ala Met Ser
            20                  25                  30

<210>  9
<211>  34
<212>  PRT
<213>  Homo sapiens

<220>
<221>  misc_feature
<222>  (1)..(1)
<223>  Xaa is pyroglutamic acid

<400>  9

Xaa Ala Ser Asn Cys Phe Ala Ile Arg His Phe Glu Asn Lys Phe Ala
1               5                   10                  15

Val Glu Thr Leu Ile Cys Ser Arg Thr Val Lys Lys Asn Ile Ile Glu
            20                  25                  30

Glu Asn

<210>  10
<211>  34
<212>  PRT
<213>  Homo sapiens

<220>
<221>  misc_feature
<222>  (1)..(1)
<223>  Xaa is pyroglutamic acid

<400>  10

Xaa Ala Ser Asn Cys Phe Ala Ile Arg His Phe Glu Asn Lys Phe Ala
1               5                   10                  15

Val Glu Thr Leu Ile Cys Phe Asn Leu Phe Leu Asn Ser Gln Glu Lys
            20                  25                  30

His Tyr

<210>  11

<211>  4
<212>  PRT
<213>  Homo sapiens

<400>  11

Trp Asn Ser Phe
1

<210>  12
<211>  10
<212>  PRT
<213>  Homo sapiens

<400>  12

Asp Leu Pro Asn Tyr Asn Trp Asn Ser Phe
1               5                   10

## Description

### Field of the Invention

The present invention relates to Kisspeptin-like peptides which are suitable for use in therapy.

### Background of the Invention

The progressive loss of cognitive function, characteristics of Alzheimer's disease, is accompanied by two disease-associated pathological changes in the brain. One of the changes involves formation of plaques in the space between neuronal cells, composed primarily of Amyloid-β (Aβ-a protein in the brain). The second change involves that deformation of another brain protein, tau, which normally regulates the function of neurons. The deformation of tau results in the formation of Neurofibrillary Tangles (NFTs), which are insoluble, twisted fibres that build up inside the neuronal cells.

The Aß protein is generated from the Amyloid-ß Precursor Protein (AßPP) with the major forms Aß 1-42 and Aß 1-40 and the N-terminally truncated P3 peptides (Aß 17-40 and Aß 17-42) being generated by alternative enzymatic processing of AßPP. The C-terminally extended forms of Aß (Aß 1-42 and Aß 17-42) show increased ability to form fibrils and are thought to have a causative action in the neurodegeneration seen in Alzheimer's disease (Tabaton et al., Exp. Neurol. 221: 18-25 (2010): Mattson, Physiol. Rev. 77: 1081-1132 (1997); Rosenblum, J. Neuropath. Exp. Neurol. 58: 575-581 (1999)).

All the major forms of Aß contain a functional neurotoxic domain (Aß 25-35) and mediate their neurotoxicity by binding to the intracellular Aß-binding protein ERAB, an alcohol dehydrogenase (Muirhead et al., Biochem. J. 426: 255-270 (2010): Yan et al., J. Biol. Chem. 274: 2145-2156 (1999); Yanker et al., Science 250: 279-282 (1990)). The major forms of Aß also inhibit hydrogen peroxide breakdown by the antioxidant enzyme catalase, an effect that involves a direct high affinity binding reaction (Milton, Biochem. J. 344: 293-296 (1999)). The Aß 25-35 region shows sequence similarity to the Diabetes-associated Islet Amyloid Polypeptide (IAPP; also known as Amylin) and the Creutzfeldt-Jakob disease associated prion protein (PrP). The Aß peptides can kill both neuronal and non-neuronal cells, a property shared with IAPP and PrP. Fibril forming fragments of all three peptides are also capable of: modifying mitochondrial function; causing oxidative stress; modifying cell cycle processes; forming membrane channels; activating apoptotic cascades; altering intracellular calcium balance; altering glucose metabolism; and modifying metabolic processes (Kawahara et al., J. Biol. Chem. 275: 14077-14083 (2000); Lim et al., FEBS Lett. 582, 2188-2194 (2008); Lim et al., Proteomics 10: 1621-1633 (2010)).

The Aß 31-35 peptide is the shortest cytotoxic form of Aß, inhibits catalase and inhibits binding of Aß peptides to catalase (Milton, Biochem. J. 344: 293-296 (1999); Milton & Harris, Micron 40: 800-810 (2009)). Both catalase and antibodies specific to this region prevent Aß cytotoxicity, suggesting that compounds which specifically bind Aß 31-35 may be of therapeutic value in the treatment of Alzheimer's disease.

The Aß 16-20 region has been shown to be responsible for binding to ERAB (Milton et al., Neuroreport 12: 22561-2566 (2001); Oppermann et al., FEBS Lett. 451: 238-242 (1999)). Antibodies which block Aß binding to ERAB prevent Aß 1-42 cytotoxicity, suggesting that compounds which specifically bind Aß 16-20 may also antagonise actions of Aß.

It has also been proposed that an alteration in the structure of the Aß protein may be an important determinant of cytotoxicity (Selkoe, Nature 399: A23-A31 (1999)). Chronic inhibition of phosphatases can cause Alzheimer's-like pathology (Arendt et al., Neurobiol. Aging; 19:3-13 (1998)) suggesting that Alzheimer's pathology may be due to an imbalance of kinase/phosphatase levels. The appearance of Aß plaques in such animal models suggests that phosphorylation actions are crucial in the biochemical processes underlying Aß plaque formation. The ability of cyclin-dependent kinase inhibitors to prevent Aß toxicity also suggests a key role for such kinases in the toxic actions of Aß (Giovanni et al., J. Biol. Chem; 274:19011-6 (1999); Alvarez et al FEBS Lett; 459: 421-6 (1999)). These enzymes specifically phosphorylate serine and threonine residues within substrates and play roles in cell division and apoptosis.

The Aß peptide has been suggested to cause both neurotoxicity (Laurén et al., Nature 457: 1128-1132 (2009)) and memory deficits (Gimbel et al., J. Neurosci. 30(18): 6367-6374 (2010)) via interaction with the cellular PrP (Laurén et al., Nature 457: 1128-1132 (2009)), which is required for PrP toxicity (Radford & Mallucci, Curr. Issues Mol. Biol. 12: 119-128 (2009)) and also Amylin receptors (Jhamandas & MacTavish, J. Neurosci. 24: 5579-5584 (2004)), which are required for IAPP toxicity (Jhamandas et al., J. Neurophysiol. 89: 2923-2930 (2003)). As such, compounds that prevent these interactions may be neuroprotective. Protective actions of Aß (Plant et al., J. Neurosci. 23: 5531-5535 (2003)), IAPP (Cantarella et al., Pharmacol. Res. 56: 27-34 (2007)) and PrP peptides (Rial et al., Neuroscience 164: 896-907 (2009)) have been suggested. As such, selection of compounds for prevention of toxicity without modifying these beneficial protective actions is preferable.

Kisspeptin (also known as metastin) is a peptide derived from the Kiss1 gene and a ligand for the GPR-54 or Kisspeptin receptor (Kotani et al., J. Biol. Chem. 276: 34631-34636 (2001); Muir et al., J Biol Chem 276: 28969-28975 (2001); Ohtaki et al., Nature 411: 613-617 (2001)). The Kisspeptin peptides range in size from 10 to 54 amino acids, with the 10 amino acid form the smallest biologically active derivative and considered the physiologically active form (Gutiérrez-Pascual et al., Mol. Pharmacol. 76: 58-67 (2009); Bilban et al., J. Cell Sci. 117: 1319-1328 (2004)). The main Kisspeptin forms isolated from tissues and body fluids are the 54 amino acid form Kisspeptin-54; the 14 amino acid form Kisspeptin-14; the 13 amino acid form Kisspeptin-13 and the decapeptide Kisspeptin-10 (Kotani et al., J. Biol. Chem. 276: 34631-34636 (2001); Bilban et al., J. Cell Sci. 117: 1319-1328 (2004)). Among the biological activities of Kisspeptins mediated via interaction with the GPR-54 receptor are: effects on cell proliferation; prevention of metastasis; activation of luteinizing-hormone-releasing hormone (also known as LHRH, gonadotropin-releasing hormone or GnRH) secretion; effects on cognition; regulation of neurogenesis; a role in the pathogenesis of epilepsy; increases aldosterone production; stimulation of glucose-induced insulin secretion; and actions as potent vasoconstrictors (Oakley et al., Endocrine Reviews 30: 713-743 (2009)). Kissorphins are post-translationally modified products of the Kiss1 gene, which have been processed by enzymes such as matrix-metalloprotease (MMP) enzymes to remove the Leu-Arg-Phe motif at the C-terminus (Takino et al., Oncogene, 22: 4617-4626 (2003)) and then alpha-amidated (McDonald et al., Cell Tissue Res., 280: 159-170 (1995)) to give a Phe-NH2 group at the newly created C-terminus. The Kissorphin peptides can be derived from any of the naturally occurring forms of Kisspeptin including, but not restricted to, Kisspeptin-54, Kisspeptin-14, Kisspeptin-13 and Kisspeptin-10. These sequences have no biological actions mediated by the GPR-54 receptor (also known as Kisspeptin receptor: Takino et al., Oncogene, 22: 4617-4626 (2003)).

### Summary of the Invention

The present invention is based on the realisation that peptides derived from human Kisspeptin 1-54 have binding affinity to Amyloid-ß and related proteins. Therefore, such peptides are useful therapeutics in the treatment of conditions associated with Amyloid fibril-forming peptides and can act to prevent fibril formation.

According to a first aspect of the invention, a peptide comprising the amino acid sequence defined herein as SEQ ID No. 2, is for use in the treatment of a condition associated with Amyloid fibril-forming peptides or proteins, wherein the peptide does not directly activate or inhibit the GPR-54 G-protein coupled receptor (also known as the Kisspeptin receptor).

According to a second aspect of the invention, an antibody having affinity against a peptide defined above, is for use in the treatment of a condition associated with Amyloid fibril-forming peptides.

According to a third aspect of the invention a composition comprises an antibody or peptide as defined above, in a pharmaceutically-acceptable diluent.

According to a fourth aspect of the invention, an *in vitro* method for determining whether a subject has, or is predisposed to having, a disorder associated with Amyloid formation, comprising treating a sample from the subject with a peptide or antibody as defined above, to determine whether the sample comprises Amyloid fibril-forming peptides.

According to a fifth aspect of the invention, there is an assay for determining whether a subject has, or is predisposed to having, an increased or decreased level of the peptide in blood, body fluid or tissues.

According to a sixth aspect, there is a peptide comprising the amino acid sequence defined herein as SEQ ID No. 11, for use in the treatment of a condition associated with Aß toxicity.

The peptides of the invention are advantageous as they have therapeutic utility and, as they correspond to a natural human sequence, will not stimulate immunorejection. The peptides are modified so that they do not directly activate or inhibit the GPR-54 G-protein coupled receptor (also known as the Kisspeptin receptor), and so do not exhibit a conventional Kisspeptin activity of activating the release of Luteinizing-hormone-releasing hormone (also known as LHRH, Gonadotropin-releasing hormone or GnRH).

### Brief Description of the Figures

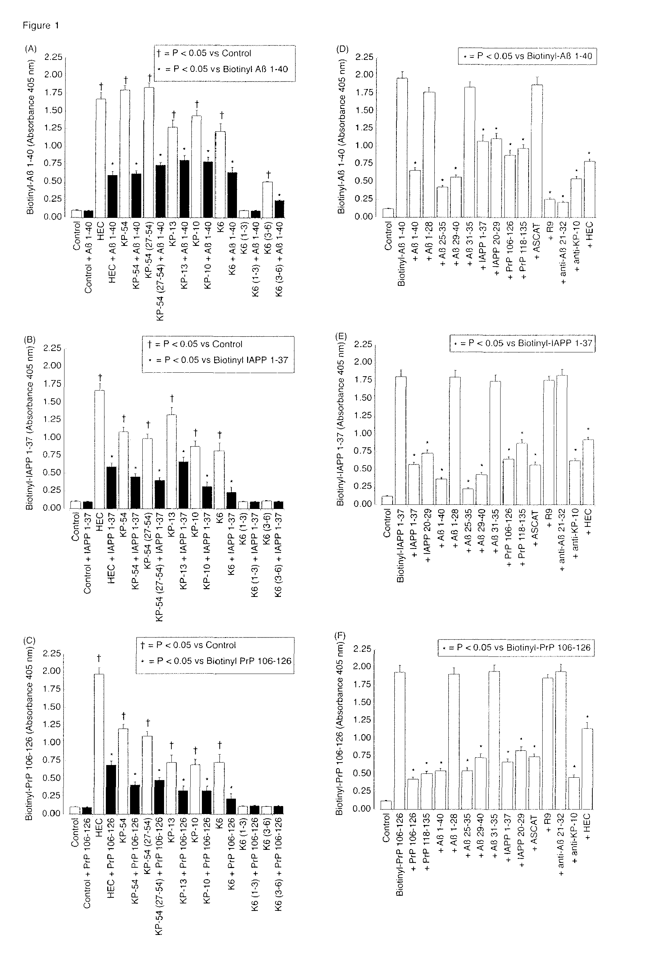

Figure 1 is a graph illustrating the binding of Kisspeptin-54, Kisspeptin-54 (27-54), Kisspeptin-13, Kisspeptin-10 and Kissorphin-6 peptides to (A) biotinyl-Aß 1-40; (B) biotinyl-IAPP 1-37 and (C) biotinyl-PrP 106-126. Inhibition of (D) biotinyl-Aß 1-40; (E) biotinyl-IAPP 1-37 and (F) biotinyl-PrP 106-126 binding to Kissorphin-6 by amyloid peptides, amyloid antagonist peptides, anti-Aß antibody, anti-Kisspeptin antibody and human erythrocyte catalase is also shown.

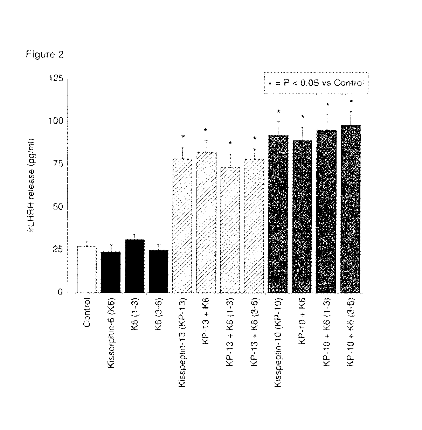

Figure 2 is a graph illustrating the release of immunoreactive Luteinizing-hormone-releasing hormone (LHRH) from the mouse GT1 hypothalamic cell line in response to Kissorphin-6, Kissorphin-6 (1-3), Kissorphin-6 (3-6), Kisspeptin-13 and Kisspeptin-10 plus combinations of Kisspeptin and Kissorphin peptides.

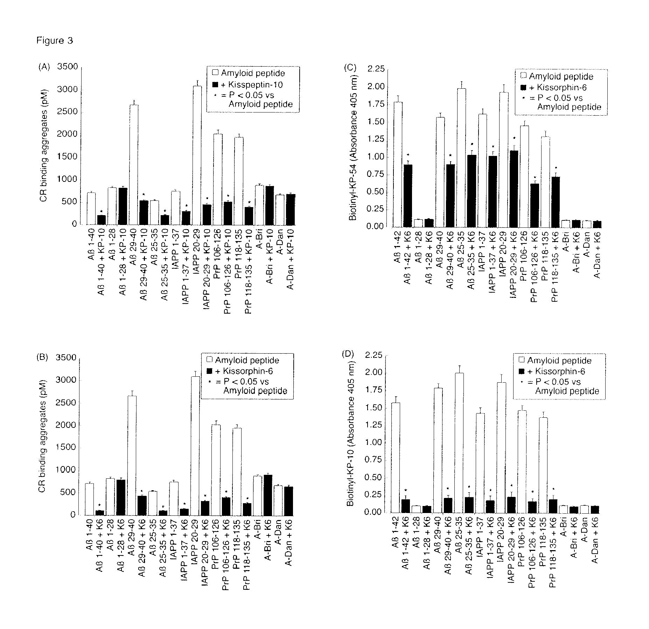

Figure 3 is a graph illustrating the effects of (A) Kisspeptin-10 and (B) Kissorphin-6 peptides on the aggregation of Aß 1-42, IAPP 1-37, PrP 106-126, PrP 118-135, Amyloid-Bri and Amyloid-Dan. The binding of (C) biotinyl-Kisspeptin-54 and (D) biotinyl-Kisspeptin-10 peptides to Aß 1-42, Aß 25-35, IAPP 1-37, IAPP 20-29, PrP 106-126, PrP 118-135, Amyloid-Bri and Amyloid-Dan fibrils are also shown.

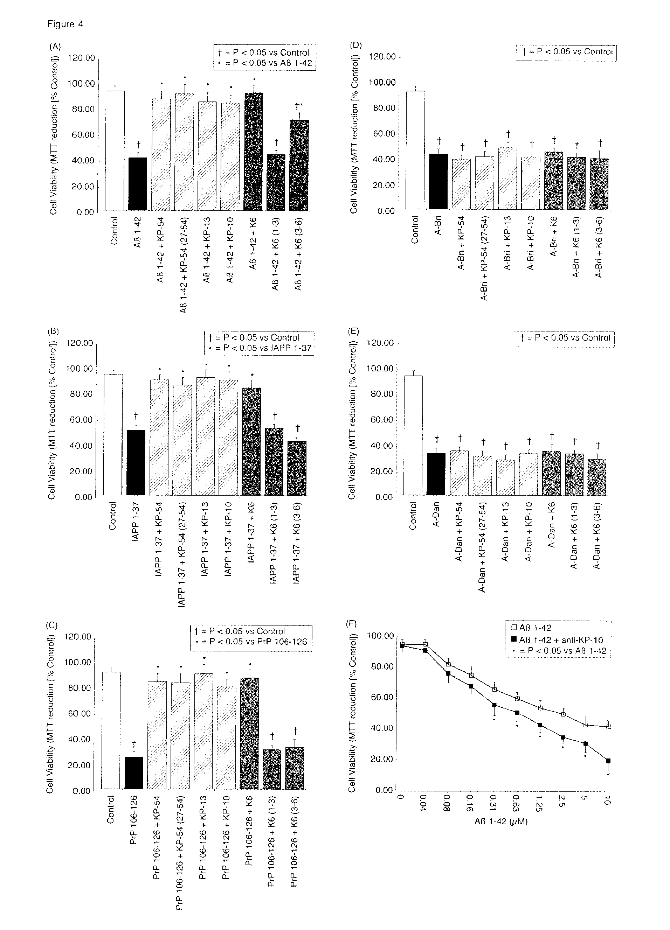

Figure 4 is a graph illustrating the role of Kisspeptin and Kissorphin peptides in amyloid peptide toxicity. The effects of Kisspeptin-54, Kisspeptin-54 (27-54), Kisspeptin-13, Kisspeptin-10, Kissorphin-6, Kissorphin-6 (3-6) and Kissorphin-6 (1-3 amide) peptides on (A) Aß 1-42 cytotoxicity; (B) IAPP 1-37 cytotoxicity; (C) PrP 106-126 cytotoxicity (D) Amyloid-Bri cytotoxicity and (E) Amyloid-Dan cytotoxicity in human SH-SY-5Y neuroblastoma cells. The effects of an anti-Kisspeptin-10 antibody on Aß 1-42 cytotoxicity (F) in human SH-SY-5Y neuroblastoma cells is also shown.

### Detailed Description of the Invention

The present invention is based on the realisation that peptides derived from Kisspeptin 1-54 have the ability to bind to Amyloid fibril-forming peptides, and therefore can act to block the biological actions plus the formation of fibrils, thereby preventing the progression of associated disease states.

The peptides can inhibit the generation of the Aβ peptide from its precursor protein, can inhibit the aggregation of Aβ into neurotoxic aggregates, can block the neurotoxic actions of Aβ peptide forms and can enhance the clearance of the Aβ peptide. The peptides of the invention have been found to bind to a number of Amyloid fibril forming peptides. Such peptides include Diabetes-associated Islet Amyloid Polypeptide (IAPP; also known as Amylin), Creutzfeldt-Jakob disease associated prion protein (PrP) and the Alzheimer's-associated Amyloid-ß peptide (Aß), but not the Amyloid-Bri or Amyloid-Dan peptides associated with two non-Aß cerebral amyloidoses familial British and Danish dementias (Ghiso et al., Brain Pathol. 16: 71-79 (2006)). The peptides of the invention do not share the Kisspeptin biological activity of stimulating the release of luteinizing-hormone-releasing hormone (also known as LHRH, gonadotropin-releasing hormone or GnRH) via activation of the GPR-54 G-protein coupled receptor (also known as the Kisspeptin receptor) and are not antagonists of this activity. The peptides of the invention inhibit the aggregation of Aß, IAPP and PrP into toxic aggregates and can prevent the toxicity of Aß, IAPP or PrP.

The Aß peptide is central to Alzheimer's disease (Panza et al., Aging Clin. Exp. Res. 21: 386-406 (2009)) but is also linked to Cerebral amyloid angiopathy (Smith & Greenberg, Stroke 40: 2601-2606 (2009)), Down's syndrome (Lott et al., Curr. Alzheimer Res. 3:521-528 (2006)) plus brain ischemia and brain trauma (Hiltunen et al., J. Alzheimers Dis. 18: 401-412 (2009)). Since Aß can enhance the aggregation of other pathological proteins there is potential for peptides of the invention to also be used in diseases associated with protein aggregates and deposits. For example Aß enhances α-synuclein aggregation plus pathological events, which are involved in the development of Lewy-body diseases (Masliah et al., Proc Natl Acad Sci USA 98: 12245-12250 (2001)). Likewise increased levels or deposits of Aß are also found in a range of Neurodegenerative diseases including Parkinson's (Burack et al., Neurology 74: 77-84 (2010)), Frontotemporal lobar degeneration and amyotrophic lateral sclerosis (Steinacker et al., J. Neural. Transm. 116: 1169-1178 (2009)) plus Huntington's (Mollenhauer et al., J. Neurol. Neurosurg. Psychiatry 77: 1201-1203 (2006)) suggesting the peptides of the invention could be used in these and other conditions in which Aß is implicated.

The IAPP peptide is linked to Diabetes Mellitus (Höppener & Lips, Int. J. Biochem. Cell Biol. 38, 726-736 (2006)) and also obesity (Roth et al., Arch. Neurol. 66, 306-310 (2009)). The PrP protein is linked to transmissible spongiform encephalopathies (TSEs) such as Creutzfeldt-Jakob disease (Aguzzi & Calella, Physiol. Rev. 89: 1105-1152, (2009)) and Gerstmann-Sträussler-Scheinker disease (Hsiao et al., Nat. Genet. 1: 68-71(1992)).

Pathology in diseases such as the Creutzfeldt-Jakob disease include Aß deposits alongside PrP deposits in the brain (Paquet et al., Acta Neuropathol 116: 567-573 (2008); Debatin et al., Neurodegenerative Dis 5: 347-354 (2008)). Likewise Diabetes also includes Aß deposits alongside IAPP in the pancreas (Miklossy et al., Neurobiol Aging. doi:10.1016/ j.neurobiolaging. 2008.08.019 (2008)) suggesting that drugs targeting combinations of Aß, IAPP and PrP may be more effective in many disorders.

Accordingly, the peptides of the invention can be used to treat disorders associated with Amyloid peptides. Such disorders include, but are not restricted to: Alzheimer's; Cerebral amyloid angiopathy; Down's syndrome; brain ischemia; brain trauma; Lewy-body diseases; Parkinson's; Frontotemporal lobar degeneration; Amyotrophic lateral sclerosis; Huntington's; Diabetes Mellitus; obesity; Transmissible spongiform encephalopathies (TSEs); and Creutzfeldt-Jakob disease.

The Kisspeptin 1-54 amino acid sequence required to bind to the Amyloid peptides is shown as SEQ ID No 1.

The minimum amino acid sequence from Kisspeptin required to bind to the Amyloid peptides is shown as SEQ ID No. 2.

SEQ ID No. 2: Tyr-Asn-Trp-Asn-Ser-Phe

This represents amino acids 45-50 on the full-length Kisspeptin sequence.

The peptides of the invention will comprise at least the sequence shown as SEQ ID No. 2. Larger peptides are also envisaged. If the larger peptides also comprise further Kisspeptin amino acid sequences they should be modified so that the sequence does not elicit normal Kisspeptin biological activity; i.e. the peptide will not activate or inhibit the GPR-54 or Kisspeptin receptor. This can be achieved in various ways, including, but not limited to the omission of the Gly-Leu-Arg-Phe C-terminal amino acid segment of the natural Kisspeptin sequence, i.e. amino acid residues 51-54 of SEQ ID No. 1.

In a separate embodiment, it has been found that a shorter sequence, identified herein as SEQ ID No.11, can be used in the treatment of a condition of AP toxicity. Therefore, the context of such disorders, the peptide will comprise at least SEQ ID No.11.

In preferred embodiments, the peptides for use in the invention consist of the following amino acid sequences:
SEQ ID No. 4: Leu-Pro-Asn-Tyr-Asn-Trp-Asn-Ser-Phe-Gly-Leu-Arg-Phe-NH₂ (KP42-54)
SEQ ID No. 5: Tyr-Asn-Trp-Asn-Ser-Phe-Gly-Leu-Arg-Phe-NH₂ (KP45-54)
SEQ ID No. 6: Asp-Leu-Pro-Asn-Tyr-Asn-Trp-Asn-Ser-Phe-NH₂ (KP41-50)

The peptides of the invention have the ability to bind to Aß1-42.

For the avoidance of doubt, reference to the Aß 1-42 region means the amino acid numbering for the conventional human Aß protein, shown as SEQ ID No. 3. Reference to the IAPP 1-37 region means the amino acid numbering for the conventional human IAPP protein, shown as SEQ ID No. 7. Reference to the PrP 106-135 region means the amino acid numbering for the conventional human PrP 106-135 protein fragment, shown as SEQ ID No. 8. Reference to Amyloid-Bri means the conventional human Amyloid-Bri protein shown in SEQ ID No. 9. Reference to Amyloid-Dan means the conventional human Amyloid-Dan protein shown in SEQ ID No. 10.

The binding of a peptide (or fragment) to the endogenous Aß 1-42, IAPP 1-37, PrP 106-135, Amyloid-Bri or Amyloid-Dan peptides (or fragments thereof) may be determined as shown in the Examples, and using techniques disclosed in Milton, Biochem. J. 344: 293-296 (1999), the content of which is incorporated herein by reference.

The peptides bind with a dissociation constant (Kd) of less than 50 µM, preferably less than 10 µM.

Provided the sequence shown as SEQ ID No. 2 is present, the peptides of the invention may also include sequences that are homologous to sequences on Kisspeptin 1-54.

The term "homologue" is used herein in two separate contexts. The peptides of the invention will comprise at least the sequence of SEQ ID No. 2, but may have additional sequences that share the same hydropathy profile as the remaining Kisspeptin sequence. This may be determined by analysing the peptide sequence and evaluating what alternative amino acids could be used as a replacement based on hydropathic character. Table 1 groups together those amino acids with a similar hydropathic character and which can be substituted for an amino acid specified in the peptide sequence.

**Table 1**

| Amino acid | Acceptable substitutions |
|---|---|
| Alanine (Ala) | Arg, Gly, Pro, Ser, Thr |
| Arginine (Arg) | Cys, Gly, Ser, Thr, Trp |
| Asparagine (Asn) | Asp, Gln, Glu,, His, Lys, Tyr |
| Aspartic acid (Asp) | Asn, Gln, Glu,, His, Lys, Tyr |
| Cysteine (Cys) | Arg, Gly, Ser, Trp |
| Glutamic Acid (Glu) | Asn, Asp, Gln, Lys, |
| Glutamine (Gln) | Asn, Asp, Glu, His, Lys, Tyr |
| Glycine (Gly) | Ala, Arg, Cys, Ser, Thr, Trp, |
| Histidine (His) | Asn, Asp, Gln, Tyr |
| Isoleucine (Ile) | Leu, Met, Val |
| Leucine (Leu) | Ile, Phe, Val |
| Lysine (Lys) | Asn, Asp, Gln, Glu |
| Methionine (Met) | Ile, Val |
| Phenylalanine (Phe) | Leu, |
| Proline (Pro) | Ala, Ser, Thr |
| Serine (Ser) | Ala, Arg, Cys, Gly, Pro, Thr, Trp |
| Threonine (Thr) | Ala, Arg, Gly, Pro, Ser |
| Tryptophan (Trp) | Arg, Cys, Gly, Ser |
| Tyrosine (Tyr) | Asn, Asp, Gln, His |
| Valine (Val) | Ile, Leu, Met |

The term "homologue" is also used to refer to peptides that share levels of sequence identity or similarity. Levels of identity or similarity between amino acid sequences can be calculated using known methods. Publicly available computer based methods include BLASTP, BLASTN and FASTA (Atschul et al., Nucleic Acids Res., 25: 3389-3402 (1997)), the BLASTX program available from NCBI, and the GAP program from Genetics Computer Group, Madison WI.

The levels of identity and similarity referred to herein are based on the use of the BLASTP program. All BLAST searches were carried out using the Standard protein-protein BLAST (blastp) on the NCBI web site (www.ncbi.nlm.nih.gov/BLAST) with the BLOSUM62 matrix and Gap Costs of 11 for Existence and 1 for Extension. The statistical significance threshold for reporting matches against database sequences (E) was reset to 100 to account for the use of short peptide sequences in the search.

It is preferable if there is at least 60% sequence identity or similarity to the specified peptides, preferably 70%, more preferably 80% and most preferably greater than 90%, e.g. at least 95%. The peptides should retain the ability to bind to the Aß protein and will comprise at least SEQ ID No. 2.

Synthetic amino acid derivatives may also be used. For example, the shifting of substitutents within an amino acid residue, from a C atom to a N atom, to produce a peptide having greater resistance to proteolysis, and other modifications, are known and are included within the scope of this invention. The importance of amino acids with ring structures in SEQ ID No. 2 is noted and substitutions within the sequence of the Tyr or Trp with amino acids containing an alternative ring structure from one of His, Pro, Phe, Tyr or Phe are included within the scope of this invention.

Peptides of the invention may be synthesised using conventional methods known in the art and can be obtained to order from commercial sources. Peptide synthesis methods are also disclosed in Chan & White, Fmoc Solid Phase Peptide Synthesis: A Practical Approach (2000).

Alternatively, the peptides may be produced using recombinant DNA technology. This can be accomplished using techniques known to those skilled in the art. For example, the DNA sequence to be expressed can be inserted into an appropriate expression vector that contains the necessary regulatory apparatus, e.g. promoters, enhancers etc, to enable expression to occur. The DNA sequence may also be a synthetic polynucleotide. The expression vector can then be inserted into an appropriate host cell, to enable expression to occur. Suitable methods are disclosed in Sambrook et al, Molecular Cloning, A Laboratory Manual (1989), and Ausubel et al, Current Protocols in Molecular Biology (1995), John Wiley & Sons, Inc., the content of each being incorporated herein by reference.

The peptides used in the invention may also comprise a signal sequence, which aids transport of the peptide *in vivo.* For example, the signal sequence may be provided to aid transport of the peptide across the blood brain barrier. Suitable signal sequences will be apparent to the skilled person.

The peptides of the invention may also be modified to prevent their degradation in *vivo.* For example the peptides may be modified to be resistant to peptidases present in the body. Suitable technologies to achieve this will be apparent to the skilled person.

Compounds that bind specifically to the peptide of the invention may also be useful in the diagnosis of a disease associated with Amyloid fibril-forming peptides. Novel antibodies may be raised using known antibody production techniques. For example, a peptide of the present invention, acting as an antigen, may be administered to an animal to produce an antibody-rich serum. This "antiserum" can be purified, to remove unwanted antibody molecules, by, for example, affinity fractionation. Monoclonal antibodies may also be raised by, for example, animal or *in vitro* immunisation techniques and fusion of antigen-exposed spleen cells to a myeloma cell line to produce hybridoma cell lines that secrete antibody. By screening hybridoma cell lines with a peptide of the invention, specific antibody-producing cell lines may be established.

The term "antibody" includes, but is not limited to, polyclonal, monoclonal, chimeric, single chain, Fab fragments and fragments produced by a Fab expression library. Such fragments include fragments of whole antibodies which retain their binding activity for a target substance, Fv, F(ab') and F(ab')2 fragments, as well as single chain antibodies (ScFv), fusion proteins and other synthetic proteins which comprise the antigen-binding site of the antibody.

In a preferred embodiment, a peptide fragment defined herein as SEQ ID No. 2, is used to raise antibodies that are specific for the Kissorphin peptide. The techniques of phage display or ribosome display, both of which are conventional in the art, may be used to select those antibodies with high affinity, preferably greater than 10⁻³ M, more preferably greater than 10⁻⁵ or 10⁻⁶ M. The antibodies may be useful in therapy or diagnostic assays.

The peptide or antibodies derived therefrom can be used in the diagnostic tests to evaluate the risk of disease, or to provide a prognosis.

It will be evident to the skilled person how the diagnostic test can be carried out. In general, a sample from a subject can be treated with a peptide of the invention or an antibody thereto, and detection of a binding event can be measured and quantified with reference to a control sample.

The peptide or antibody can be detectably labelled, so that a binding event can be monitored. The label can, for example be a fluorophore or biotin label, covalently bound to the peptide or antibody.

A diagnostic assay where the levels of Kissorphin and Kisspeptin peptides are determined in biological fluids or tissue samples are determined can be used for both diagnosis and therapy monitoring. Techniques such as HPLC or column chromatography can be used to separate different Kissorphin and Kisspeptin forms, which can be detected using standard methods (Lim (ed), HPLC of Small Molecules: a Practical Approach. Oxford: IRL Press; (1986); Hutton & Siddle (eds), Peptide Hormone Secretion: a Practical Approach. Oxford: IRL Press; (1990)). The assay can also use antibodies or other binding agents, capable of specific recognition of the Kissorphin and Kisspeptin peptides for detection and standard immunoassay techniques for level determination (Hutton & Siddle (eds), Peptide Hormone Secretion: a Practical Approach. Oxford: IRL Press; (1990)).

A sample from a patient (blood sample, tissue sample etc.) can be used to detect whether an amyloid peptide, or fragment thereof, that binds the peptide of the invention such as Aß, IAPP or PrP is present. The amyloid peptide, or fragment thereof, that binds the peptide of the invention such as Aß, IAPP or PrP can be detected, for example, by the use of an antibody that has specificity for a peptide of the invention or by use of a peptide of the invention.

The peptides of the invention may also be used in assays to identify therapeutic molecules that can target an amyloid peptide, or fragment thereof, that binds the peptide of the invention such as Aß, IAPP or PrP. For example, combinatorial chemistry can be used to develop target therapeutic molecules, which are then screened for activity. The target molecules can be brought into contact with a protein of the invention. If the presence of the target molecule results in binding affinity, then it may be a potential therapeutic candidate.

The peptides or antibodies for use in the invention may be formulated, in a therapeutically effective amount, in any suitable pharmaceutically acceptable, diluent or excipient. Acceptable corners, diluents and excipients are well known to the skilled person and are also described in Remington's Pharmaceutical Sciences, Mack Publishing Co (A.R. Gennaro edit. 1985). The choice of a corner diluent or excipient can be selected based on the intended route of administration, etc.

The pharmaceutical composition may be for human or animal usage. The dosage to be administered to a patient depends on the route of administration, the nature of the formulation and the severity of the condition. Intravenous infusion of Kisspeptin-54 over the dose range up to 0.01 mg/kg body weight has been shown to activate endogenous LH in humans (Dhillo et al., J. Clin. Endocrinol. Metab. 90: 6609-6615 (2005)). A Kisspeptin receptor antagonist administered via intracerebroventricular injection in rats at a dose equivalent to 0.03 mg/kg body weight inhibited the actions of Kisspeptin (Roseweir et al., J. Neurosci., 29: 3920 - 3929 (2009)). Suitable doses of the peptide of the invention are likely to be in the range of 0.01 -30 mg/kg body weight, preferably from 0.01-10 mg/kg body weight and more preferably 0.01-1 mg/kg body weight.

The pharmaceutical composition can be administered via any convenient route. Typically, the composition will be administered parenterally, in which case the composition is in an injectable form, for delivery via the intravenous, intramuscular or subcutaneous route.

### Example

### Amyloid peptide binding to Kisspeptin and Kissorphin peptides

N-terminally Biotinylated Kisspeptin-54 (SEQ ID No. 1), Kisspeptin-10 (SEQ ID No. 12), Aß 1-42 (SEQ ID No. 3), Aß 1-40, PrP 106-126 (SEQ ID No. 8) and IAPP 1-37 (SEQ ID No. 7) were purchased from Bachem or Alpha Diagnostics. ELISA plates were coated with either Kisspeptin-54, Kisspeptin-54 (27-54), Kisspeptin-13, Kisspeptin-10, Kissorphin-6 (SEQ ID No. 2), Aß 1-42, Aß 25-35, IAPP 1-37, IAPP 20-29, PrP 106-126, PrP 118-135, Amyloid-Bri (SEQ ID No. 9) or Amyloid-Dan peptides (SEQ ID No. 10), (1 µg ml-1) in carbonate buffer and unoccupied sites blocked with 0.2% (w/v) casein. Biotinylated peptides (200 pM) were incubated alone, with control peptides, with Kissorphin peptides peptides, Kisspeptin peptides or with unlabelled forms of the respective amyloid peptides in 50 mM TRIS (containing 0.1% BSA and 0.1% Triton X-100) at 4°C for 16 hours. After washing to remove unbound material an alkaline phosphatase polymer-streptavidin conjugate (Sigma, Dorset, UK) was added and incubated at 24°C for 2 hours. After washing to remove unbound material p-nitrophenylphosphate substrate was added and absorbance at 405 nm determined. Affinity constants were determined by incubating Kissorphin-6 coated plates with biotinylated peptides (200 pM) plus unlabelled peptides over a range of concentrations (0-100 nM) and detection of bound peptides by ELISA.

Scatchard analysis (Friguet, B. et al., J. Imm. Meth. 77: 305-319 (1985)) was performed using the following equation: A0 / (A0 - A) = 1 + KD / a0 and plotting v [(A0 - A) / A0] against v / a, where A0 = absorbance in absence of unlabelled peptide, A = absorbance in presence of unlabelled peptide, a0 = total concentration of unlabelled peptide and a = concentration of unlabelled peptide added. The KD was equal to - 1 / slope of v against v / a.

The biotinylated Aß 1-42 (Figure 1A), IAPP 1-37 (Figure 1B) and PrP 106-126 (Figure 1 C) all showed significant binding to Kisspeptin-54, Kisspeptin-54 (27-54), Kisspeptin-13, Kisspeptin-10 and Kissorphin-6 coated plates. Binding of biotinylated Aß 1-42 to the Kissorphin-6 peptide (Figure 1 D) was inhibited by unlabelled amyloid peptides, the anti-amyloid nonapeptide R9 (Milton & Harris, Micron 40: 800-810 (2009)), an anti-Aß monoclonal antibody ND1 (Milton & Harris, TSWJ 10: 879-893 (2010)), an anti-Kisspeptin-10 antibody, and human erythrocyte catalase (HEC: Milton, Biochem. J. 344: 293-296 (1999)).

Binding of biotinylated IAPP 1-37 to the Kissorphin-6 peptide (Figure 1 E) was inhibited by unlabelled amyloid peptides, a synthetic amyloid antagonist peptide ASCAT (Pro-Pro-Arg-Gly-Leu-Nle-Nle-Gly-Leu-Ser-Lys described in Milton et al., Neuroreport 12; 2561-2566 (2001)), an anti-Kisspeptin-10 antibody, and human erythrocyte catalase (HEC: Milton, Biochem. J. 344: 293-296 (1999)), the anti-Aß monoclonal antibody ND1 (Milton & Harris, TSWJ 10: 879-893 (2010)) and anti-amyloid nonapeptide R9 (Milton & Harris, Micron 40: 800-810 (2009)) had no effect of IAPP binding to Kissorphin-6. Binding of biotinylated PrP 106-126 to the Kissorphin-6 peptide (Figure 1 F) was inhibited by unlabelled amyloid peptides, the synthetic amyloid antagonist peptide ASCAT (Pro-Pro-Arg-Gly-Leu-Nle-Nle-Gly-Leu-Ser-Lys described in Milton *et al.,* Supra, an anti-Kisspeptin-10 antibody, and human erythrocyte catalase (HEC: Milton, Supra, the anti-Aß monoclonal antibody ND1 (Milton & Harris 2010, Supra, and anti-amyloid nonapeptide R9 (Milton & Harris 2009, Supra) had no effect of IAPP binding to Kissorphin-6. The Aß fragments Aß 1-28 and 31-35 had no effect on binding of biotinylated Aß, IAPP and PrP peptides whilst Aß 25-35 and 29-40 both strongly inhibited binding suggesting that residues 29 and 30, plus some of the surrounding residues may be key.

The antibody raised against the Kisspeptin-10 peptide, which also bound Kissorphin-6, was able to prevent binding of biotinylated Human Aß 1-42, Human Aß 1-40, Human PrP 106-126 and Human Amylin 1-37 to plates coated with Kissorphin-6. Similar antibodies may be used to monitor Kissorphin levels.

Affinity constants (KD) for binding to Kissorphin-6 were 0.62 ± 0.07 nM (n=5) for Aß 1-42; 0.47 ± 0.04 nM (n=5) for Aß 1-40; 11.6 ± 1.2 nM (n=5) for IAPP 1-37 and 34.2 ± 3.7 nM (n=5) for PrP 106-126 respectively. These constants are similar to those previously determined for Aß 1-42 and IAPP 1-37 binding to human erythrocyte catalase and ERAB (Milton, Biochem. J. 344: 293-296 (1999); Milton et al., Neuroreport 12: 2561-2566 (2001); Milton & Harris, TSWJ 10: 879-893 (2010)).

### Effects of Kisspeptin and Kissorphin on LHRH release

To determine if the Kisspeptin and Kissorphin sequences tested above had biological activity the release of Luteinizing-hormone-releasing hormone (LHRH) from a Kisspeptin responsive cell line was determined. The mouse LHRH secreting cell line (GT1) was cultured in Dulbecco's modified Eagle's medium (DMEM) and Ham's F-12 (ratio 1:1) containing 10% fetal bovine serum (FBS) and sodium bicarbonate 3.7 g/I, in a humidified atmosphere containing 5% CO2 in air (Al-Damluji et al., Br. J. Pharmacol. 132: 336-344 (2001)). Culture media were changed at 48 h intervals. Cells were exposed to Kisspeptin-13, Kisspeptin-10, Kissorphin-6, Kissorphin-6 (1-3 amide) or Kissorphin-6 (3-6) for 2h and release of LHRH measured by immunoassay (Quaynor et al., Mol. Endocrinol. 21: 3062-3070 (2007)).

Both Kisspeptin-13 and Kisspeptin-10 stimulated a 4-fold increase in LHRH release whilst the Kissorphin peptides had no effect. Addition of Kisspeptin-13 or Kisspeptin-10 plus Kissorphin-6, Kissorphin-6 (1-3) or Kissorphin-6 (3-6) (SEQ ID No. 11) showed that the Kissorphin peptides had no significant effect on Kisspeptin-13 or Kisspeptin-1 0 stimulated release of immunoreactive LHRH (Figure 2). These results confirmed that the Kissorphin-6 peptide sequences were neither agonists nor antagonists of LHRH release.

### Effects of Kisspeptin and Kissorphin peptides on amyloid peptide aggregation

Amyloid peptides recognised by Kisspeptin and Kissorphin peptides have the ability to aggregate in to Congo red staining fibrils and other forms all of which have been linked to pathology and toxicity in Amyloid diseases (Milton & Harris, Micron 40: 800-810 (2009); Milton & Harris, TSWJ 10: 879-893 (2010)). The Kisspeptin and Kissorphin peptides were tested for their ability to inhibit the aggregation process and their interactions with fibrillar aggregates.

Freshly prepared 50 µM solutions of amyloid peptides (Aß 1-42, IAPP 1-37, PrP 106-126, PrP 118-135, Amyloid-Bri and Amyloid-Dan) were incubated either alone or in the presence of either Kisspeptin-10 or Kissorphin-6, after dissolving in distilled water, at 37°C for 24h with constant oscillation. Following in vitro fibrillogenesis of peptides an aliquot of each test sample was prepared to give a 50 µM concentration of amyloid peptide in phosphate buffered saline (PBS). Congo red, prepared as a 200 µM stock in PBS containing 10% ethanol, was then added to give final concentration 10 µM Congo red to 9.09 µM amyloid peptide and 100 µl aliquots were added to 96 well microtitre plates. After 15 min incubation the absorbance levels at 405 and 540nm were determined. The concentration of amyloid aggregates ([Amyloidagg]) was then calculated, with correction for the pathlength of the reader used, as follows: [Amyloidagg] = 10 x ((540At/4780) - (405At/6830) - (405ACR/8620)), where 540At = absorbance of amyloid peptide + Congo red solution at 540 nm, 405At = absorbance of amyloid peptide + Congo red solution at 405 nm and 405ACR = absorbance of Congo red solution at 405 nm. Absorbance readings at 405 nm and 540 nm were taken for each amyloid peptide and the ratio of 540AAmyloid /405AAmyloid checked, where 405AAmyloid = absorbance of amyloid peptide solution at 405 nm and 540AAmyloid = absorbance of amyloid peptide solution at 540 nm (Milton & Harris, Micron 40: 800-810 (2009)). The Aß 1-42, IAPP 1-37, PrP 106-126, PrP 118-135, Amyloid-Bri and Amyloid-Dan peptides all showed significant amyloid aggregate formation. The concentrations of amyloid aggregates formed by Aß 1-42, IAPP 1-37, PrP 106-126 and PrP 118-135 were significantly reduced by treatment with Kisspeptin-10 (Figure 3A) or Kissorphin-6 (Figure 3B). The Kisspeptin-10 and Kissorphin-6 peptides had no significant effect on the amyloid aggregate formation by either Amyloid-Bri or Amyloid-Dan peptides. Previous studies have shown that Kisspeptin-10 can form aggregates, but that this requires the addition of enhancers such as heparin (Nielsen et al., Biopolymers doi: 10.1002/bip.21434 (2010)), in the system used here Kisspeptin-10 and Kissorphin-6 showed no detectable aggregate formation.

Biotinylated Kisspeptin-54 (Figure 3C) and Kisspeptin-10 (Figure 3D) both bound plates coated with Aß 1-42, Aß 25-35, IAPP 1-37, IAPP 20-29, PrP 106-126 and PrP 118-135 fibrils. No binding to Amyloid-Bri or Amyloid-Dan fibrils was observed. These results confirm that the binding to amyloid peptides could take place in situations where both monomeric, oligomeric and fibrillar forms are present.

### Effects of Kisspeptin and Kissorphin peptides on amyloid neurotoxicity

The human neuroblastoma SH-SY5Y cell line was routinely grown in a 1:1 mixture of HAM's F12 and Eagle's minimal essential medium containing 1% non-essential amino acids and supplemented with 10% fetal calf serum and antibiotics (penicillin and streptomycin; 1% each) in a 5% CO2 humidified incubator at 37°C. The cells were passaged when confluent and medium was replaced every 2-3 days. Cells were used 4-10 days after plating. Cultures were differentiated for 7 days with 10 mM retinoic acid (Milton, Open Enzyme Inhib. J. 1; 34-41 (2008)).

For cytotoxicity experiments 5 x 103 cells were incubated in 96 well dishes in 100 µl culture medium and test substances for 24 hours. Cell viability was determined by trypan blue dye exclusion with at least 100 cells counted per well or by MTT reduction (Behl et al., Cell 77; 817-827 (1994)). After incubation with test substances MTT (10 µl: 12mM stock) was added and cells incubated for a further 4 hours. Cell lysis buffer [100 µl/well; 20% (v/v) SDS, 50 % (v/v) N,N-dimethylformamide, pH 4.7] was added and after repeated pipetting to lyse cells the MTT formazan product formation was determined by measurement of absorbance change at 570 nm. Control levels in the absence of test substances were taken as 100% and the absorbance in the presence of cells lysed with Triton X-100 at the start of the incubation period with test substances taken as 0% acid (Milton, Open Enzyme Inhib. J. 1; 34-41 (2008)).

The toxicity of Aß 1-42 and Aß 25-35 peptides (Milton, Neurotoxicology 22: 767-774 (2001); Milton, Neurosci. Lett. 332: 127-130 (2002)) was significantly inhibited by addition of Kisspeptin-54, Kisspeptin-54 (27-54), Kisspeptin-13, Kisspeptin-10, Kissorphin-6 and Kissorphin-6 (3-6) peptides (Figure 4A). The Kissorphin-6 (1-3 amide) peptide had no effect on the toxicity of Aß 1-42 or Aß 25-35 peptides.

The toxicity of IAPP 1-37 and IAPP 20-29 peptides (Smith et al., J. Am. Chem. Soc. 131: 4470-4478 (2009); Tenidis et al., J. Mol. Biol. 295: 1055-1071 (2000)) was significantly inhibited by addition of Kisspeptin-54, Kisspeptin-54 (27-54), Kisspeptin-13, Kisspeptin-10 and Kissorphin-6 peptides (Figure 4B). The Kissorphin-6 (1-3 amide) and Kissorphin-6 (3-6) peptides peptide had no effect on the toxicity of IAPP 1-37 or IAPP 20-29 peptides.

The toxicity of the PrP 106-126 and PrP 118-135 peptides (Chabry et al., J. Neurosci. 23: 462-469 (2003); Henriques et al., Biochemistry 48: 4198-4208 (2009)) was significantly inhibited by addition of Kisspeptin-54, Kisspeptin-54 (27-54), Kisspeptin-13, Kisspeptin-10 and Kissorphin-6 peptides (Figure 4C). The Kissorphin-6 (1-3 amide) and Kissorphin-6 (3-6) peptides peptide had no effect on the toxicity of PrP 106-126 or PrP 118-135 peptides.

The toxicity of the Amyloid-Bri peptide (Gibson et al., Biochem Soc Trans. 33: 1111-1112 (2005)) was unaffected by addition of Kisspeptin-54, Kisspeptin-54 (27-54), Kisspeptin-13, Kisspeptin-10, Kissorphin-6, Kissorphin-6 (3-6) or Kissorphin-6 (1-3 amide) peptides (Figure 4D).

The toxicity of the Amyloid-Dan peptide (Gibson et al., Biochem Soc Trans. 33: 1111-1112 (2005)) was unaffected by addition of Kisspeptin-54, Kisspeptin-54 (27-54), Kisspeptin-13, Kisspeptin-10, Kissorphin-6, Kissorphin-6 (3-6) or Kissorphin-6 (1-3 amide) peptides (Figure 4E).

The toxicity of the Aß 1-42 peptide (Milton, Neurotoxicology 22: 767-774 (2001); Milton, Neurosci. Lett. 332: 127-130 (2002)) was significantly enhanced in the presence of an anti-Kisspeptin-10 antibody (Figure 4F) indicating a potential neuroprotective action by an endogenous Kisspeptin-like molecule.

## Claims

1. A peptide comprising the amino acid sequence defined herein as SEQ ID No. 2, for use in the treatment of a condition associated with Amyloid fibril-forming peptides, wherein the peptide does not directly activate or inhibit the GPR-54 G-protein coupled receptor.

2. A peptide according to claim 1, wherein the condition is Alzheimer's disease.

3. A peptide according to claim 1, wherein the condition is Creutzfeldt-Jakob disease.

4. A peptide according to claim 1, wherein the condition is diabetes mellitus.

5. A peptide comprising the amino acid sequence defined herein as SEQ ID No. 11, for use in the treatment of a condition associated with Aß toxicity.

6. A peptide according to any of claims 1 to 5, wherein the peptide comprises a first part that consists of amino acid sequence defined herein as SEQ ID No. 2 and optionally a second part which comprises a signal sequence.

7. A peptide according to any of claims 1 to 5, wherein the peptide comprises a first part which consists of any of the amino acid sequences defined herein as SEQ ID Nos. 4 to 6, and optionally a second part which comprises a signal sequence.

8. A peptide as defined in any of claims 1, 6 and 7, comprising a detectable label.

9. A peptide according to claim 8, wherein the label is a fluorophore or biotin.

10. An antibody having affinity against a peptide defined in claim 1, 6, 7 or 8 for use in the treatment of a condition associated with Amyloid fibril-forming peptides.

11. An antibody according to claim 10, wherein the condition is as defined in any of claims 2 to 4.

12. A composition comprising a peptide as defined in any of claims 1, 5, 6, 7, 8 or 9, in a pharmaceutically-acceptable diluent.

13. An *in vitro* method for determining whether a subject has, or is predisposed to having, a disorder associated with Amyloid formation, comprising treating a sample from the subject with a peptide as defined in any of claims 1, 5, 6, 7 or 8 or an antibody as defined in claim 10, to determine whether the sample comprises Amyloid fibril-forming peptides.

14. A method according to claim 13, wherein the peptide or antibody is detectably-labelled.
